# EUROPEAN PATENT APPLICATION

(11) **EP 3 932 933 A1**
(43) Date of publication of application: **05.01.2022**
(21) Application number: 20762296.0
(22) Date of filing: 03.02.2020
(51) Int. Cl.: C07K 1/08

(54) **METHOD FOR PRODUCING AMIDE**

(30) Priority: 28.02.2019 JP 2019036628
(71) Applicant: Tokyo Institute of Technology, Tokyo 152-8550 (JP); Yokogawa Electric Corporation, Tokyo 180-8750 (JP)
(72) Inventor: FUSE Shinichiro, Tokyo 152-8550 (JP); MASUDA Koshiro, Tokyo 152-8550 (JP); OTAKE Yuma, Tokyo 152-8550 (JP); NAKAMURA Hiroyuki, Tokyo 152-8550 (JP)
(74) Representative: Grünecker Patent- und Rechtsanwälte PartG mbB
(86) International application number: PCT/JP2020/003908
(87) International publication number: WO 2020/175023

(57) **Abstract**

A method for producing an amide, including
mixing a first composition containing a carboxylic acid or a carboxylic acid active species, and an organic solvent and a second composition containing an amine having at least one carboxyl group and water and causing the carboxylic acid or the carboxylic acid active species to react with the amine to obtain an amide.

## Description

### [Technical Field]

The present invention relates to a method for producing an amide.

Priority is claimed on Japanese Patent Application No. 2019-036628, filed February 28, 2019, the content of which is incorporated herein by reference.

### [Background Art]

In peptide synthesis, a carboxyl group of an amino acid is activated and reacted with an amino group of an amino acid, a coupling reaction is caused to form amide bonds, these operations are repeated, and thus the amino acid is sequentially extended. Several methods are known as methods of activating carboxyl groups. There are a method of synthesizing peptides while minimizing isomerization and production of byproducts using a condensing agent having a low degree of activation and a method of synthesizing peptides using an activation agent in a short time.

Examples of a method of activating the carboxyl group using a highly active activation agent include acid chloride methods and acid anhydride methods. Compared with an activation method using a condensing agent having a low degree of activation, these acid chloride methods and acid anhydride methods have advantages such as low unit price, a small amount of produced byproducts derived from an activation agent, and the like because the structure of the activation agent is simpler.

Regarding the amino acid used as a raw material in peptide synthesis, a protected amino acid in which a functional group is protected by a protecting group is generally used in order to prevent reactions other than a desired reaction. In the peptide synthesis, in the related art, a protected amino acid in which a carboxyl group is protected is used as an amino acid for an amino group which is reacted.

However, when a protected amino acid is used, since a deprotection process is required for each coupling reaction, both the deprotection and the coupling reaction need to be repeated, which makes the process complicated. Therefore, a production method in which an unprotected amino acid can be used is required.

Non-Patent Literature 1 to 6 describe a peptide synthesis method including a process in which a carboxylic acid is reacted with an activation agent to produce an active species as an intermediate, and the active species is coupled with an unprotected amino acid in which a carboxyl group is unprotected. Examples of active species include activated acyl compounds such as a benzotriazole amide, a pentafluorophenol ester, an acid azide, an acid fluoride, a p-nitrophenol ester, a succinimide ester, a mixed acid anhydride, and acyloxyhydroxybenzoamide.

### [Citation List]

### [Non-Patent Literatures]

[Non-Patent Literature 1]
   A. R. Katritzky, P. Angrish, E. Todadze, I. Ghiviriga, Bioorg. Med. Chem. Lett., Vol. 17 (2007) 6000-6002.
[Non-Patent Literature 2]
   S. B. Tsogoeva, S. B. Jagtap, Z. A. Ardemasova, V. N. Kalikhevich, Eur. J. Org. Chem. (2004) 4014-4019.
[Non-Patent Literature 3]
   P. Gagnon, X. Huang, E. Therrien, J. W. Keillor, Tetrahedron Lett., Vol. 43 (2002) 7717-7719.
[Non-Patent Literature 4]
   G. W. Anderson, J. E. Zimmerman, F. M. Callahan, J. Am. Chem. Soc., Vol. 86 (1964) 1839-1842.
[Non-Patent Literature 5]
   Y.-H. Chen, P.-H. Sung, K. Sung, Amino Acids, Vol. 38 (2010) 839-845.
[Non-Patent Literature 6]
   D. S. Kemp, S-W. Wang, J. Rebekjr., R. C. Mollan, C. Banquer, G. Subramanyam, Tetrahedron, Vol. 30 (1974) 3955-3967.

### [Summary of Invention]

### [Technical Problem]

Generally, activated acyl compounds (active species) are unlikely to dissolve in water and unprotected amino acids are unlikely to dissolve in organic solvents. Therefore, to perform peptide synthesis, an active species production reaction is caused in an organic phase, and a coupling reaction with an unprotected amino acid is caused at an interface of a two-phase system including an organic phase and an aqueous phase. In addition, to perform peptide synthesis, the obtained active species may be separated from the organic phase, and the coupling reaction with an unprotected amino acid may be caused in water or an organic solvent, or a mixed solvent thereof. However, in the method in which a coupling reaction is caused at an interface of the two-phase system, the main reaction is delayed, and there are problems such as a decrease in the yield due to side reactions and the progress of epimerization. The method of separating active species has problems such as an increase in the number of separation steps.

Although methods such as using a reagent for dissolving an unprotected amino acid in an organic phase have been devised, all methods have problems such as an increase in the number of processes and a need for a reagent purifying process.

The present invention has been made in order to address the above problems, and an object of the present invention is to provide a method for producing an amide in which an amine having at least one carboxyl group such as an unprotected amino acid can be used as a raw material, reaction efficiency is favorable, and side reactions such as epimer production are unlikely to occur.

### [Solution to Problem]

The inventors conducted extensive studies in order to address the above problems and as a result, found that the above problems can be addressed when an organic phase in which an active species is produced and an aqueous phase including an amine having at least one carboxyl group are mixed to cause a reaction, and completed the present invention.

Specifically, the present invention has the following aspects.

(1) A method for producing an amide, including
   mixing a first composition containing a carboxylic acid or a carboxylic acid active species, and an organic solvent and a second composition containing an amine having at least one carboxyl group and water and causing the carboxylic acid or the carboxylic acid active species to react with the amine to obtain an amide.
(2) The method for producing an amide according to the above-described (1), including
   before the mixing, activating the carboxylic acid to obtain the carboxylic acid active species.
(3) The method for producing an amide according to the above-described (1) or (2), including
   mixing a first composition containing a product obtained by reacting a mixture obtained by mixing a carboxylic acid and an activation agent, and an organic solvent and a second composition containing an amine having at least one carboxyl group and water and causing the product to react with the amine to obtain an amide.
(4) The method for producing an amide according to the above-described (3), including
   before the mixing, reacting a mixture obtained by mixing a carboxylic acid and an activation agent.
(5) The method for producing an amide according to any one of the above-described (1) to (4),
   wherein the first composition contains the carboxylic acid active species, and the carboxylic acid active species is any one or more selected from the group consisting of a mixed acid anhydride, a mixed carbonate anhydride, an acid azide, an acid halide, a benzotriazole amide, a pentafluorophenol ester, a p-nitrophenol ester, and a succinimide ester.
(6) The method for producing an amide according to the above-described (5),
   wherein the mixed acid anhydride is synthesized by any one or more selected from the group consisting of isopropyl chloroformate, isobutyl chloroformate, ethyl chloroformate, 2,4-dimethyl-3-pentyl chloroformate, isopropyl bromoformate, isobutyl bromoformate, ethyl bromoformate, and 2,4-dimethyl-3-pentyl bromoformate.
(7) The method for producing an amide according to any one of the above-described (3) to (6),
   wherein the activation agent is a halogenated formate ester, and
   wherein the first composition contains a product obtained by reacting a mixture obtained by mixing a carboxylic acid, the halogenated formate ester, and a second base that activates the halogenated formate ester, and an organic solvent.
(8) The method for producing an amide according to the above-described (7),
   wherein the second base that activates the halogenated formate ester is any one or more selected from the group consisting of tertiary amines, 4-methylmorpholine, pyridine, pyridine derivatives, imidazole, imidazole derivatives and 1,4-diazabicyclo[2,2,2]octane.
(9) The method for producing an amide according to the above-described (1) or (2), including
   mixing a first composition containing a product obtained by reacting a mixture obtained by mixing a first carboxylic acid and a second carboxylic acid, and an organic solvent, and a second composition containing an amine having at least one carboxyl group and water, and causing the product to react with the amine to obtain an amide.
(10) The method for producing an amide according to the above-described (9), including
   before the mixing, reacting a mixture obtained by mixing a first carboxylic acid and a second carboxylic acid.
(11) The method for producing an amide according to the above-described (10),
   wherein the first composition contains a product obtained by mixing the first carboxylic acid, the second carboxylic acid, and phosgene or a phosgene equivalent that decomposes in a reaction system and produces phosgene and dehydrating and condensing the carboxylic acids, and the organic solvent.
(12) The method for producing an amide according to any one of the above-described (1) to (11),
   wherein the carboxylic acid is an amino acid or an amino acid derivative.
(13) The method for producing an amide according to any one of the above-described (1) to (12),
   wherein the amine is an amino acid or an amino acid derivative.
(14) The method for producing an amide according to any one of the above-described (1) to (13),
   wherein the amine is an unprotected amino acid in which functional groups in the side chain are protected, and amino groups and carboxyl groups are not protected or modified.
(15) The method for producing an amide according to any one of the above-described (12) to (14),
   wherein the amino acids or amino acid derivatives are 20 types of amino acids or derivatives thereof that constitute proteins and are encoded by genetic information.
(16) The method for producing an amide according to any one of the above-described (1) to (15),
   wherein the first composition and the second composition are mixed in a distribution system reaction device.
(17) The method for producing an amide according to the above-described (16),
   wherein, in the distribution system reaction device, a liquid feeding rate of the first composition is 2 mL/min or more, and a liquid feeding rate of the second composition is 2 mL/min or more.
(18) The method for producing an amide according to any one of the above-described (1) to (17),
   wherein the second composition or a mixture containing the first composition and the second composition contains a base.
(19) The method for producing an amide according to any one of the above-described (1) to (18),
   wherein, in the second composition, a proportion of the content of water with respect to 100 volume% of the component corresponding to the solvent is 10 to 100 volume%.
(20) The method for producing an amide according to any one of the above-described (1) to (19),
   wherein the organic solvent is compatible with water.

### [Advantageous Effects of Invention]

According to the present invention, it is possible to provide a method for producing an amide, in which an amine having at least one carboxyl group can be used as a raw material, reaction efficiency is favorable and side reactions such as epimer production are unlikely to occur.

### [Brief Description of Drawings]

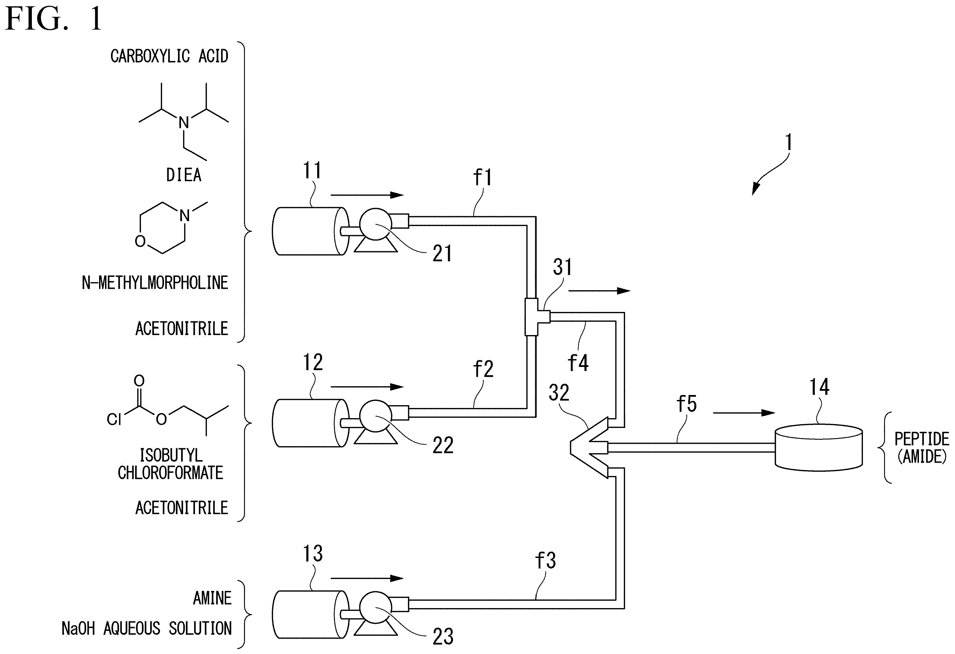

Fig. 1 is a schematic view showing a schematic configuration of a distribution system reaction device 1.

### [Description of Embodiments]

A method for producing an amide according to an embodiment of the present invention will be described below.

### «Method for producing amide»

The method for producing an amide according to the embodiment is a method including mixing a first composition containing a carboxylic acid or a carboxylic acid active species, and an organic solvent and a second composition containing an amine having at least one carboxyl group and water and causing the carboxylic acid or the carboxylic acid active species to react with the amine to obtain an amide.

The method for producing an amide according to the embodiment may include, before the mixing, activating the carboxylic acid to obtain a carboxylic acid active species.

In addition, the method for producing an amide according to the embodiment may be a method including mixing a first composition containing a product obtained by reacting a mixture obtained by mixing a first carboxylic acid and a second carboxylic acid or a product obtained by reacting a mixture obtained by mixing a carboxylic acid and an activation agent, and an organic solvent and a second composition containing an amine having at least one carboxyl group and water, and causing the product to react with the amine to obtain an amide.

The method for producing an amide according to the embodiment may include, before the mixing, reacting a mixture obtained by mixing a first carboxylic acid and a second carboxylic acid or a mixture obtained by mixing a carboxylic acid and an activation agent.

Here, the term "mixing" as used herein refers to an operation of adding substances such as raw materials to the reaction system, and when these are mixed in the reaction system, raw materials and the like may be changed to substances different from those before addition.

The mixture obtained by mixing a first carboxylic acid and a second carboxylic acid or the mixture obtained by mixing a carboxylic acid and an activation agent may contain the organic solvent in advance.

### <First embodiment>

A method for producing an amide according to the present embodiment includes the following Processes 1-1 to 2-1.

Hereinafter, Process 1-1 and the following Process 1-2 and Process 1-2-1 will be abbreviated as "Process 1" sometimes. Hereinafter, Process 2-1 and the following Process 2-2 and Process 2-2-1 will be abbreviated as "Process 2" sometimes.

The method for producing an amide according to the present embodiment is a method in which an acid anhydride which is a carboxylic acid active species is used as the product.

Process 1-1: process in which carboxylic acids are dehydrated and condensed to obtain a first composition containing an acid anhydride, and an organic solvent.
Process 2-1: process in which the first composition obtained in Process 1-1 and a second composition containing an amine having at least one carboxyl group and water are mixed, and the acid anhydride is reacted with the amine to produce an amide.

Hereinafter, the above processes will be described. Here, the reactions in the method for producing an amide according to the present invention are not limited to reactions exemplified in the following processes.

### <Process 1-1>

Process 1-1 is a process in which carboxylic acids are dehydrated and condensed to obtain a first composition containing an acid anhydride, and an organic solvent.

The first composition may contain a product obtained by reacting a mixture obtained by mixing a carboxylic acid and phosgene or a phosgene equivalent that decomposes in a reaction system and produces phosgene, and an organic solvent.

The carboxylic acids are reacted with phosgene or a phosgene equivalent that decomposes in a reaction system and produces phosgene, the carboxylic acids are dehydrated and condensed, and an acid anhydride can be obtained.

The carboxylic acid may be any having a carboxyl group at the end of a molecule and may be represented by the following General Formula (1). 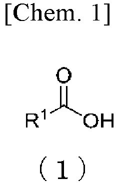 (in the formula, R¹ represents a hydrogen atom or a monovalent organic group)

The carboxylic acid may be deprotonated into carboxylate ions and may be represented by the following General Formula (1i). 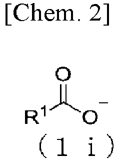 (in the formula, R¹ represents a hydrogen atom or a monovalent organic group)

Deprotonation of the carboxylic acids can be achieved, for example, by placing the carboxylic acids in the presence of a base having low nucleophilicity such as N,N-diisopropylethylamine (DIEA) in the reaction system.

The presence of a base means, for example, in a solvent to which a base is added. The first composition may contain a product obtained by reacting a mixture obtained by mixing the first carboxylic acid, the second carboxylic acid, a base having low nucleophilicity such as DIEA, and phosgene or a phosgene equivalent that decomposes in a reaction system and produces phosgene, and an organic solvent. The mixture may contain the organic solvent in advance. The type of the base is not particularly limited as long as it allows the carboxylic acid to be deprotonated in the reaction system.

In Process 1-1 in the method for producing an amide according to the embodiment, carboxylic acids represented by the following General Formula (1) and carboxylic acids represented by the following General Formula (1)' are dehydrated and condensed to obtain an acid anhydride represented by the following General Formula (2). The acid anhydride can be obtained, for example, by reacting a carboxylic acid with phosgene or a phosgene equivalent that decomposes in a reaction system and produces phosgene. 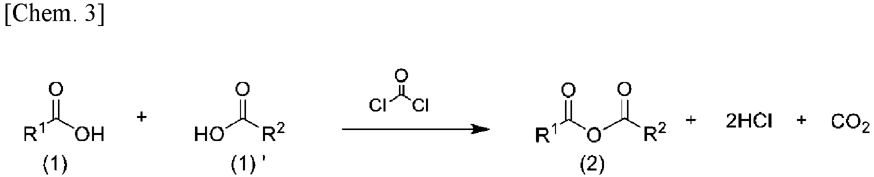 (in the formula, R¹ and R² each independently represent a hydrogen atom or a monovalent organic group)

The phosgene equivalent is a chemical that decomposes in a reaction system and produces phosgene, and can be used in substantially the same way as phosgene in a synthesis reaction. Examples of phosgene equivalents include diphosgene and triphosgene.

In the dehydration condensation, carboxylic acids of different types may be dehydrated and condensed, or carboxylic acids of the same type may be dehydrated and condensed. That is, R¹ and R² in Formulae (1) and (1)' may be the same or different from each other.

When R¹ and R² are the same, the acid anhydride represented by General Formula (2) is a symmetric acid anhydride. When R¹ and R² are the same, counter anions of an amide produced in Process 2-1 to be described below are the same as carboxylate ions before activation. Counter anions may self-react with an amide, but if counter anions are the same as carboxylate ions before they become an acid anhydride, if self-reacted, the product becomes the same as the asymmetric acid anhydride.

Therefore, when R¹ and R² are the same, there are advantages that the type of amide obtained in the reaction system is uniform, and it is easy to systematically obtain a desired type of the product.

The carboxylic acid is preferably an amino acid or an amino acid derivative. The carboxylic acid here includes a carboxylic acid that is a precursor of a carboxylic acid active species. The amino acid is preferably α-amino acid. In addition, generally, since amino acids constituting peptides or proteins in a living body are an L type, the amino acids may be an L type. The α-amino acid may be a compound represented by the following General Formula (1-1). 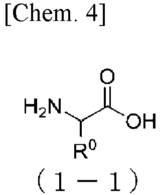 (in the formula, R⁰ represents a side chain of an amino acid)

The amino acids may be 20 types of amino acids that constitute peptides or proteins in a living body and are encoded by genetic information. Examples of these amino acids include alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine. In addition, the amino acid may be a type of amino acid such as cysteine that is not encoded by genetic information.

For example, R⁰ in Formula (1-1) is "-CH₃" when the amino acid is alanine, "-H" when the amino acid is glycine, "-CH(CH₃)₂" when the amino acid is valine, and "-CH(CH₃)CH₂CH₃" when the amino acid is isoleucine. The same applies to other amino acids.

When Formulae (1) and (1)' are amino acids, -R¹ and -R² each may be-CH(R⁰)NH₂.

The amino acid may be α-amino acid, and may be, for example, an β-amino acid such as β-alanine.

The carboxylic acid may be an amino acid derivative. The amino acid derivative may be a compound having substantially the same properties as an amino acid, and may be a natural type that occurs naturally or a type which has modifications such as alternation, addition, or substitution of a functional group different from those of the natural type.

As an example of a case having substantially the same properties as an amino acid, a case in which amino acid derivatives can be incorporated into an enzyme that uses an amino acid as a substrate and a case in which amino acid derivatives can be bound to molecules that bind to an amino acid may be exemplified.

Examples of amino acid derivatives include those in which one or more hydrogen atoms or groups in the amino acid are substituted with other groups (substituents). As an example of amino acid derivatives, a protected amino acid in which a functional group is protected by a protecting group may be exemplified. The protecting group has a function of inactivating a reactive functional group. It is possible to deprotect the protecting group and return the protected functional group to its unprotected state. Here, the fact that the functional group is protected means that atoms constituting the functional group are substituted with a protecting group. Examples of sites protected by a protecting group include any one or more sites selected from the group consisting of amino groups, carboxyl groups, and side chains. One or two or more functional groups contained in the side chain may be protected by a protecting group. In Process 1, it is preferable that amino groups and/or functional groups in the side chain be protected so that the reaction of the reactive functional group other than carboxyl groups is prevented.

The type of the protecting group is not particularly limited, and can be appropriately selected depending on the type of the functional group to be protected. Examples of amino group protecting group include carbamate-based, sulfonamide-based, acyl-based, and alkyl-based protecting groups, but the present invention is not limited thereto.

Examples of carbamate-based protecting groups include 2-benzyloxycarbonyl groups (sometimes abbreviated as -Z or -Cbz), tert-butoxycarbonyl groups (sometimes abbreviated as -Boc), allyloxycarbonyl groups (sometimes abbreviated as -Alloc), 2,2,2-trichloroethoxycarbonyl groups (sometimes abbreviated as -Troc), 2-(trimethylsilyl)ethoxycarbonyl groups (sometimes abbreviated as -Teoc), 9-fluorenylmethyloxycarbonyl groups (sometimes abbreviated as -Fmoc), p-nitrobenzyloxycarbonyl groups (sometimes abbreviated as -Z(NO₂)), and p-biphenylisopropyloxycarbonyl groups (sometimes abbreviated as -Bpoc).

Examples of sulfonamide-based protecting groups include p-toluenesulfonyl groups (sometimes abbreviated as -Ts or -Tos), 2-nitrobenzenesulfonyl groups (sometimes abbreviated as -Ns), 2,2,4,6,7-pentamethyldihydrobenzofuran-5-sulfonyl (sometimes abbreviated as -Pbf), 2,2,5,7,8-pentamethylchromane-6-sulfonyl (sometimes abbreviated as -Pmc), and 1,2-dimethylindole-3-sulfonyl (sometimes abbreviated as - MIS).

The first composition contains an organic solvent. The organic solvent is an organic compound that can dissolve a carboxylic acid or a carboxylic acid active species and is preferably a liquid at 1 atom and 25°C. When the first composition contains an organic solvent, a carboxylic acid or a carboxylic acid active species can be favorably dissolved. In particular, amino acid derivatives and peptides having a protecting group and their active species can also be favorably dissolved. The organic solvent is preferably a solvent that is compatible with water, preferably a polar solvent, and more preferably an aprotic polar solvent in consideration of the reactivity. When a solvent having compatibility with water is used, the mixed state with water contained in the second composition becomes favorable, and the reaction efficiency can be improved.

Examples of organic solvents in the first composition include acetonitrile, N,N-dimethylformamide (DMF), tetrahydrofuran (THF), and 1,4-dioxane.

A proportion of the content of the organic solvent with respect to 100 mass% of the first composition is preferably 10 mass% or more, more preferably 10 to 99 mass%, still more preferably 40 to 95 mass%, and most preferably 60 to 90 mass%. When the first composition contains an organic solvent in the above proportion, the reaction can proceed favorably. In particular, when a distribution system reaction device is used, if the organic solvent is contained in the above proportion, the composition that passes through the flow path of the device is distributed well and thus the reaction can proceed favorably.

The organic solvent is preferably a liquid at 1 atm and 25°C because it is suitable for use in the distribution system reaction device.

In the first composition, the proportion of the content of the organic solvent with respect to 100 volume% of the component corresponding to the solvent is preferably 50 volume% or more, more preferably 50 to 100 volume%, and still more preferably 80 to 100 volume%.

### <Process 2-1>

Process 2-1 is a process in which the first composition obtained in Process 1-1 and a second composition containing an amine having at least one carboxyl group and water are mixed, and the acid anhydride is reacted with the amine to produce an amide.

In Process 2-1 in the method for producing an amide according to the embodiment, an acid anhydride represented by the following General Formula (2) and an amine represented by the following General Formula (6) are reacted to obtain an amide represented by the following General Formula (7).

In such a process, it is preferable to react the carboxylic acid or the carboxylic acid active species with the amine under basic conditions. The basic conditions mean, for example, in a solvent to which a base (B) (where the amine is excluded) is added. For example, the mixture containing the first composition and the second composition may contain a base (B), and for example, the second composition may contain a base (B). It is considered that the inclusion of the base (B) in the reaction system improves the reactivity of the amine as a nucleophilic agent. 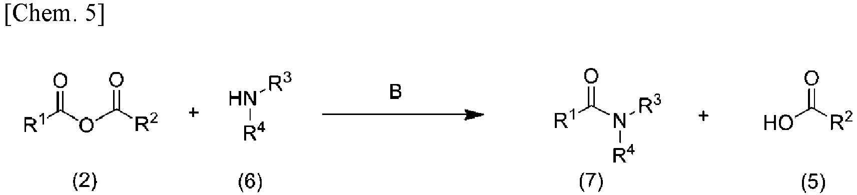 (in the formula, R¹, R², R³ and R⁴ each independently represent a hydrogen atom or a monovalent organic group)

Examples of bases (B) include alkali metal hydroxides such as lithium hydroxide, sodium hydroxide, potassium hydroxide, and cesium hydroxide; alkaline earth metal hydroxides such as calcium hydroxide and barium hydroxide; alkali metal carbonates or bicarbonates such as sodium carbonate, sodium bicarbonate, potassium carbonate, potassium bicarbonate, and cesium carbonate; and quaternary ammonium hydroxides such as tetramethylammonium hydroxide and tetrabutylammonium hydroxide.

The base (B) is preferably any one or more selected from the group consisting of potassium hydroxide, cesium hydroxide, calcium hydroxide, barium hydroxide, sodium carbonate, sodium bicarbonate, potassium carbonate, potassium bicarbonate, cesium carbonate, tetramethylammonium hydroxide, and tetrabutylammonium hydroxide.

The amine is preferably an amino acid or an amino acid derivative.

Examples of amino acids and amino acid derivatives include those exemplified as the carboxylic acid.

When Formula (6) represents an amino acid, -R³ and -R⁴ may be, for example,-H and -CH(R⁰)COOH.

As an example of amino acid derivatives, a protected amino acid in which a functional group is protected by a protecting group may be exemplified. Examples of sites protected by a protecting group include any one or more sites selected from the group consisting of amino groups, carboxyl groups, and side chains as long as the protected amino acid has at least one carboxyl group. One or two or more functional groups contained in the side chain may be protected by a protecting group.

The amine has at least one carboxyl group.

When the amine is an amino acid, at least one carboxyl group among carboxyl groups of the amino acid (including carboxyl groups that can be contained in the side chain of amino acids) is not protected and is a free carboxyl group.

When the amine is an amino acid, it is preferable that carboxyl groups of the amino acid (where, this does not include carboxyl groups that can be contained in the side chain of amino acids) be unprotected. That is, carboxyl groups forming the main chain part of the peptide are preferably unprotected.

In the amine, only functional groups in the side chain may be protected so that the reaction of reactive functional groups other than amino group is prevented.

The amine may be an unprotected amino acid in which functional groups in the side chain are protected, and amino groups and carboxyl groups forming the main chain part of the peptide are unprotected.

The amine may be an unprotected amino acid that is not protected and modified at any of sites of amino groups, carboxyl groups, and side chains.

Examples of amino acids include 20 types of amino acids that constitute proteins and are encoded by genetic information or derivatives thereof having at least one carboxyl group.

The amine is of 20 types of amino acids that constitute proteins and are encoded by genetic information, and may be an unprotected amino acid in which functional groups in the side chain are protected, and amino groups and carboxyl groups forming the main chain part of the peptide are unprotected, or an unprotected amino acid that is not protected and modified at any of sites of amino groups, carboxyl groups, and side chains.

The type of the protecting group is not particularly limited, and can be appropriately selected depending on the type of the functional group to be protected. Carboxyl groups may be protected by simply being neutralized in the form of salts, but are generally protected in the form of esters. Examples of esters include benzyl esters (sometimes abbreviated as Bn or BZ1) in addition to alkyl esters such as methyl and ethyl esters, but the present invention is not limited thereto.

The second composition contains water. When the second composition contains water, an amine having at least one carboxyl group such as an amino acid in which a carboxyl group of an amino acid is unprotected can also be favorably dissolved.

The second composition may further contain a solvent other than water, and may further contain an organic solvent. Examples of organic solvents include those exemplified in the first composition. In addition, the second composition may contain the same type of organic solvent as the organic solvent contained in the first composition.

A proportion of the content of water with respect to 100 mass% of the second composition may be 20 mass% or more, 20 to 99 mass%, 30 to 99 mass%, or 30 to 60 mass%. When water in such a proportion is contained, the reaction can proceed favorably. In particular, when the second composition or a mixture containing the first composition and the second composition contains a base, if water in a proportion of the lower limit value or more is contained, dissolution of the base proceeds, and the reaction efficiency is further improved. In addition, when a distribution system reaction device is used, if water in a proportion of the above lower limit value or more is contained, distributability of the composition in the device is improved and the reaction efficiency is further improved.

In the second composition, a proportion of the content of water with respect to 100 volume% of the component corresponding to the solvent may be 10 volume% or more, 10 to 100 volume%, 15 to 80 volume amount%, or 20 to 60 volume%. When water in such a proportion is contained, the reaction can proceed more favorably.

For the same reason, in the second composition, a proportion of the content of the organic solvent with respect to 100 volume% of the component corresponding to the solvent may be 0 to 90 volume%, 20 to 85 volume amount%, or 40 to 80 volume%.

In the mixture containing the first composition and the second composition, a proportion of the content of water with respect to 100 volume% of the component corresponding to the solvent may be 5 volume% or more, 5 to 60 volume%, 10 to 50 volume%, or 20 to 40 volume%. When water in such a proportion is contained, an amine having at least one carboxyl group can also be favorably dissolved, and the reaction can proceed favorably. In addition, when water in a proportion of the above upper limit value or less is contained, this is preferable because hydrolysis of the carboxylic acid active species can be restricted.

In the mixture containing the first composition and the second composition, a proportion of the content of the organic solvent with respect to 100 volume% of the component corresponding to the solvent may be 10 volume% or more, 40 to 95 volume%, 50 to 90 volume%, or 60 to 80 volume%. Even if the organic solvent in such a proportion is contained, the reaction can proceed favorably according to the method for producing an amide of the embodiment.

The mixture containing the first composition and the second composition contains water and an organic solvent, and with respect to 100 volume% of the component corresponding to the solvent, a proportion of the content of water may be 5 to 60 volume% and a proportion of the content of the organic solvent may be 40 to 95 volume%, a proportion of the content of water may be 10 to 50 volume% and a proportion of the content of the organic solvent may be 50 to 90 volume%, and a proportion of the content of water may be 20 to 40 volume% and a proportion of the content of the organic solvent may be 60 to 80 volume%.

According to the method for producing an amide of the embodiment, it is possible to provide a method for producing an amide, which has favorable reaction efficiency and is less likely to cause epimer production of an amide.

In a conventional method for producing an amide, a carboxylic acid active species is produced, and then separated from the solvent, and an operation in which unprotected amino acids are reacted in water or an organic solvent or in a mixed solvent containing water and an organic solvent is then performed. In this method, an extra separation process is required and also there is a risk of epimers being produced in the process.

On the other hand, according to the method for producing an amide according to the embodiment, when a first composition containing a carboxylic acid active species and an organic solvent and a second composition containing the amine and water are mixed, it is easy to immediately react the produced highly reactive carboxylic acid active species with a desired amine, the time during which the carboxylic acid active species is in an activated state can be shortened, the reaction efficiency can be improved, and production of undesired side reaction products can be effectively minimized.

### <Second embodiment>

A method for producing an amide according to the present embodiment includes the following Processes 1-2 to 2-2.

Process 1-2: process in which a mixture obtained by mixing a carboxylic acid and an activation agent is reacted to obtain a first composition containing a carboxylic acid active species and an organic solvent.
Process 2-2: process in which the first composition obtained in Process 1-2 is mixed with a second composition containing an amine having at least one carboxyl group and water, and the carboxylic acid active species and the amine are reacted to produce an amide.

Detailed descriptions of parts having the same configuration as in the above

### <First embodiment> will be omitted.

Examples of carboxylic acids include those exemplified in the above <First embodiment>, examples of amines include those exemplified in the above <First embodiment>, and examples of bases (B) include those exemplified in the above <First embodiment>.

Examples of organic solvents in the first composition include those exemplified in the above <First embodiment>.

Examples of solvents such as water and the organic solvent in the second composition include those exemplified in the above <First embodiment>.

Here, also in Process 2-2, as in the above first embodiment, it is preferable to react the carboxylic acid or the carboxylic acid active species with the amine under basic conditions. The basic conditions mean, for example, in a solvent to which a base (B) is added. For example, the mixture containing the first composition and the second composition may contain a base (B), and for example, the second composition may contain a base (B).

### (Activation agent-carboxylic acid active species)

In the method for producing an amide according to the present embodiment, an activation agent that activates carboxyl groups can be used as the activation agent.

Examples of activation agents include a halogenated formate ester, an azidization agent, a halogenating agent, and bis(pentafluorophenyl)carbonate, chloroformic acid p-nitrophenyl. Benzotriazole, pentafluorophenol, N-hydroxysuccinimide or the like can be used as an activation agent in combination with a condensing agent such as N,N'-dicyclohexylcarbodiimide (DCC).

In the method for producing an amide according to the present embodiment, as the product, an active species such as a mixed acid anhydride, an acid azide, a carboxylic acid halide, a benzotriazole amide, a pentafluorophenol ester, a p-nitrophenol ester, and a succinimide ester, which are carboxylic acid active species, can be produced.

Regarding a case in which a mixed acid anhydride is produced as a carboxylic acid active species, a case in which a mixture obtained by mixing a carboxylic acid and a halogenated formate ester is reacted to obtain a mixed acid anhydride represented by the following General Formula (II) may be exemplified. 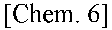 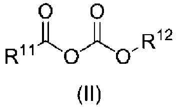 (in the formula, R¹¹ represents a hydrogen atom or a monovalent organic group, and R¹² represents a hydrogen atom or a hydrocarbon group)

An embodiment in which a mixed acid anhydride is produced as a carboxylic acid active species will be described below in detail.

Regarding a case in which an acid azide is produced as a carboxylic acid active species, a case in which a mixture obtained by mixing a carboxylic acid or a mixed acid anhydride with an azidization agent is reacted to obtain an acid azide represented by the following General Formula (II-b) may be exemplified. 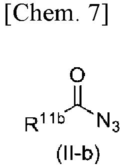 (in the formula, R^{11b} represents a hydrogen atom or a monovalent organic group)

Regarding the azidization agent, any one or more selected from the group consisting of sodium azide, trimethylsilyl azide, diphenylphosphoryl azide, tributyltin azide, and tetrabutylammonium azide may be exemplified.

Regarding a case in which a carboxylic acid halide is produced as a carboxylic acid active species, a case in which a mixture obtained by mixing an organic solvent, a carboxylic acid, and a halogenating agent is reacted to obtain a carboxylic acid halide represented by the following General Formula (II-c) may be exemplified. 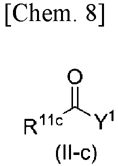 (in the formula, R^{11c} represents a hydrogen atom or a monovalent organic group, and Y¹ represents a halogen atom)

The halogen atom for Y¹ is an element belonging to Group 17 in the periodic table such as F, Cl, Br, and I, and is preferably Cl or Br.

Examples of carboxylic acid halides include carboxylic acid fluorides, carboxylic acid chlorides, and carboxylic acid bromides.

Regarding the halogenating agent, various agents may be exemplified.

Examples of halogenating agents that can be used to obtain a carboxylic acid fluoride include N,N-diethylaminosulfur trifluoride, and bis(2-methoxyethyl)aminosulfur trifluoride.

Examples of halogenating agents that can be used to obtain a carboxylic acid chloride include thionyl chloride, phosphorus trichloride, phosphorus pentachloride, sulfuryl chloride, and oxalyl chloride.

Examples of halogenating agents that can be used to obtain a carboxylic acid bromide include phosphorus tribromide.

These may be used alone or two or more thereof may be used in combination.

Regarding a case in which a benzotriazole amide is produced as a carboxylic acid active species, a case in which a mixture obtained by mixing a carboxylic acid, benzotriazole, and a condensing agent is reacted to obtain a benzotriazole amide represented by the following General Formula (II-d) may be exemplified. 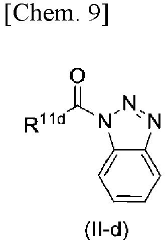 (in the formula, R^{11d} represents a hydrogen atom or a monovalent organic group)

Regarding a case in which a pentafluorophenol ester is produced as a carboxylic acid active species, a case in which a mixture obtained by mixing a carboxylic acid, pentafluorophenol and a condensing agent, or bis(pentafluorophenyl)carbonate is reacted to obtain a pentafluorophenol ester represented by the following General Formula (II-e) may be exemplified. 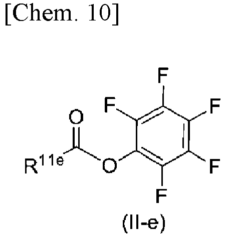 (in the formula, R^{11e} represents a hydrogen atom or a monovalent organic group)

Regarding a case in which a p-nitrophenol ester is produced as a carboxylic acid active species, a case in which a mixture obtained by mixing a carboxylic acid and chloroformic acid p-nitrophenyl is reacted to obtain a p-nitrophenol ester represented by the following General Formula (II-f) may be exemplified. In the reaction, N,N-dimethyl-4-aminopyridine (DMAP) can be additionally added, and a trimethylamine may be additionally added. 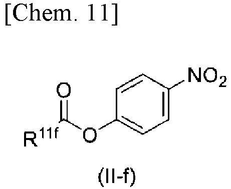 (in the formula, R^{11f} represents a hydrogen atom or a monovalent organic group)

Regarding a case in which a succinimide ester is produced as a carboxylic acid active species, a case in which a mixture obtained by mixing a carboxylic acid, N-hydroxysuccinimide, and a condensing agent is reacted to obtain a succinimide ester represented by the following General Formula (II-g) may be exemplified. 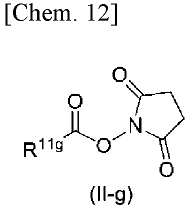 (in the formula, R^{11g} represents a hydrogen atom or a monovalent organic group)

In this manner, using the activation agent that activates carboxyl groups exemplified above, a mixture obtained by mixing a carboxylic acid and an activation agent is reacted, and thereby a first composition containing a carboxylic acid active species and an organic solvent can be obtained.

### ·Mixed acid anhydride

Hereinafter, a method using a mixed acid anhydride represented by General Formula (II) as a carboxylic acid active species will be described.

The method for producing an amide according to the present embodiment includes the following Processes 1-2-1 to 2-2-1 as the above method for producing an amide.

Process 1-2-1: process in which a mixture obtained by mixing a carboxylic acid and a halogenated formate ester is reacted to obtain a first composition containing a mixed acid anhydride, and an organic solvent.
Process 2-2-1: process in which the first composition obtained in Process 1-2-1 and a second composition containing an amine having at least one carboxyl group and water are mixed, and the mixed acid anhydride is reacted with the amine to produce an amide.

Hereinafter, the above processes will be described. Here, the reactions in the method for producing an amide according to the present invention are not limited to reactions exemplified in the following processes.

### <Process 1-2-1>

Process 1-2-1 is a process in which a mixture obtained by mixing a carboxylic acid and a halogenated formate ester is reacted to obtain a obtain a first composition containing a mixed acid anhydride, and an organic solvent.

In Process 1-2-1 in the method for producing an amide according to the embodiment, a carboxylic acid represented by the following General Formula (I) and a halogenated formate ester represented by the following General Formula (I)' are reacted to obtain a mixed acid anhydride represented by the following General Formula (II). 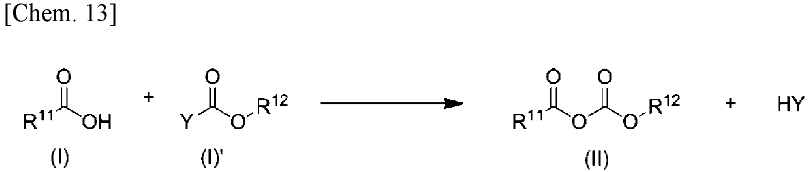 (in the formula, R¹¹ represents a hydrogen atom or a monovalent organic group, R¹² represents a hydrogen atom or a hydrocarbon group, and Y represents a halogen atom)

### Examples of carboxylic acids include those exemplified in the above <First embodiment>.

Halogenation in halogenated formate ester means that halogen atoms are bonded to carbonyl groups.

The hydrocarbon group for R¹² may be an aliphatic hydrocarbon group or an aromatic hydrocarbon group (aryl group). The aliphatic hydrocarbon group may be a saturated aliphatic hydrocarbon group (alkyl group) or an unsaturated aliphatic hydrocarbon group, and is preferably an alkyl group.

The aliphatic hydrocarbon group may have 1 to 20 carbon atoms or 1 to 15 carbon atoms.

The alkyl group may be linear, branched or cyclic. The cyclic alkyl group may be either monocyclic or polycyclic. The alkyl group may have 1 to 20 carbon atoms, 1 to 10 carbon atoms, or 1 to 5 carbon atoms.

Examples of linear or branched alkyl groups include methyl groups, ethyl groups, n-propyl groups, isopropyl groups, n-butyl groups, isobutyl groups, sec-butyl groups, tert-butyl groups, n-pentyl groups, isopentyl groups, neopentyl groups, tert-pentyl groups, 1-methylbutyl groups, n-hexyl groups, 2-methylpentyl groups, 3-methylpentyl groups, 2,2-dimethylbutyl groups, 2,3-dimethylbutyl groups, n-heptyl groups, 2-methylhexyl groups, 3-methylhexyl groups, 2,2-dimethylpentyl groups, 2,3-dimethylpentyl groups, 2,4-dimethylpentyl groups, 3,3-dimethylpentyl groups, 3-ethylpentyl groups, 2,2,3-trimethylbutyl groups, n-octyl groups, isooctyl groups, nonyl groups, decyl groups, undecylic groups, dodecyl groups, tridecylic groups, tetradecylic groups, pentadecylic groups, hexadecyl groups, heptadecyl groups, octadecyl groups, nonadecyl groups, and icosyl groups.

The halogen atom for Y is an element belonging to Group 17 in the periodic table such as F, Cl, Br, and I, and is preferably Cl or Br.

In order to more effectively minimize the side reactions, in the halogenated formate ester represented by General Formula (I)', the halogen atom for Y is Cl or Br, and the hydrocarbon group for R¹² is preferably a branched alkyl group having 1 to 5 carbon atoms and more preferably any one or more selected from the group consisting of isopropyl chloroformate, isobutyl chloroformate, ethyl chloroformate, 2,4-dimethyl-3-pentyl chloroformate, isopropyl bromoformate, isobutyl bromoformate, ethyl bromoformate, and 2,4-dimethyl-3-pentyl bromoformate.

Here, in the reaction in Process 1-2, a second base that activates the halogenated formate ester and the halogenated formate ester are reacted, and the reaction can proceed easily. Here, the activated halogenated formate ester is also included in the concept of halogenated formate ester.

The first composition may contain a product obtained by reacting a mixture obtained by mixing a carboxylic acid, the halogenated formate ester and a second base that activates the halogenated formate ester, and an organic solvent.

The second base reacts with the halogenated formate ester to produce a cationically active species and is preferably a base that more preferentially reacts with the halogenated formate ester than the mixed acid anhydride which is the product in Process 1-2-1, and is more preferably any one or more selected from the group consisting of tertiary amines, 4-methylmorpholine, pyridine, pyridine derivatives, imidazole, imidazole derivatives and 1,4-diazabicyclo[2,2,2]octane.

The pyridine derivatives may be one in which one or more hydrogen atoms of pyridine are substituted with other groups and is not particularly limited as long as it has properties of a base, and the pyridine and pyridine derivatives are preferably a compound represented by the following General Formula (3-1). 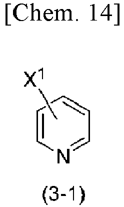 (in the formula, X¹ represents a hydrogen atom or any group selected from among the groups represented by the following Formulae (a) to (c)) 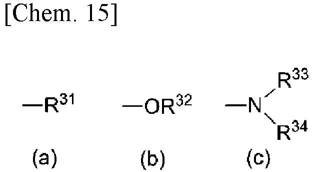 (in the formula, R³¹, R³², R³³ and R³⁴ each independently represent an alkyl group, R³³ and R³⁴ may be bonded to each other to form a ring, and one methylene group that is not directly bonded to R³³ or R³⁴ in the alkyl group may be substituted with an oxygen atom)

The alkyl group for R³¹, R³², R³³ and R³⁴ may be linear, branched or cyclic. The cyclic alkyl group may be either monocyclic or polycyclic. The alkyl group may have 1 to 20 carbon atoms, 1 to 15 carbon atoms or 1 to 10 carbon atoms.

Examples of linear or branched alkyl groups include methyl groups, ethyl groups, n-propyl groups, isopropyl groups, n-butyl groups, isobutyl groups, sec-butyl groups, tert-butyl groups, n-pentyl groups, isopentyl groups, neopentyl groups, tert-pentyl groups, 1-methylbutyl groups, n-hexyl groups, 2-methylpentyl groups, 3-methylpentyl groups, 2,2-dimethylbutyl groups, 2,3-dimethylbutyl groups, n-heptyl groups, 2-methylhexyl groups, 3-methylhexyl groups, 2,2-dimethylpentyl groups, 2,3-dimethylpentyl groups, 2,4-dimethylpentyl groups, 3,3-dimethylpentyl groups, 3-ethylpentyl groups, 2,2,3-trimethylbutyl groups, n-octyl groups, isooctyl groups, nonyl groups, decyl groups, undecylic groups, dodecyl groups, tridecylic groups, tetradecylic groups, pentadecylic groups, hexadecyl groups, heptadecyl groups, octadecyl groups, nonadecyl groups, and icosyl groups.

The compound represented by General Formula (3-1) is preferably a compound represented by the following General Formula (3-1-1). When X¹ is any group selected from among the groups represented by Formulae (a) to (c) other than a hydrogen atom, X¹ effectively functions as an electron donating group according to bonding at a relevant position, and the nucleophilicity of N atoms of a pyridine ring tends to become better. 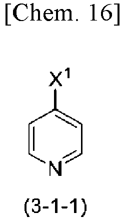 (in Formula (3-1-1), X¹ has the same meaning as X¹ in Formula (3-1))

In the compound represented by General Formula (3-1), X¹ is a group represented by Formula (c), R³³ and R³⁴ are bonded to each other to form a ring, and regarding a case in which one methylene group that is not directly bonded to R³³ or R³⁴ in the alkyl group is substituted with an oxygen atom, 4-morpholinopyridine represented by the following Formula (3-1-2) is included.

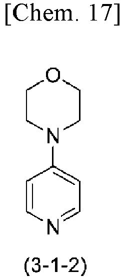

The imidazole derivative may be one in which one or more hydrogen atoms of imidazole are substituted with other groups and is not particularly limited as long as it has properties of a base, but the imidazole and imidazole derivatives are preferably a compound represented by the following General Formula (3-2). 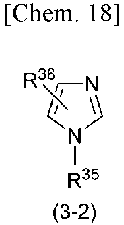 (in the formula, R³⁵ and R³⁶ each independently represent a hydrogen atom or an alkyl group)

Examples of alkyl groups for R³⁵ and R³⁶ include those exemplified as the alkyl groups for R³¹, R³², R³³ and R³⁴.

Preferable examples of imidazoles and imidazole derivatives include imidazoles and N-methylimidazole.

When R³⁶ is a hydrogen atom and R³⁵ is a methyl group, the compound represented by General Formula (3-2) includes N-methylimidazole represented by the following Formula (3-2-1).

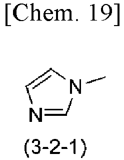

Regarding the tertiary amine, an amine in which at least one of groups bonded to N atoms of an amine is a methyl group is preferable. More preferably, two groups bonded to N atoms of an amine are methyl groups. When at least one of groups bonded to N atoms of the tertiary amine is a methyl group, the steric hindrance around the N atoms can be reduced and the reaction efficiency of the halogenated formate ester can be improved.

In view of this, examples of preferable second bases include 4-methylmorpholine, N-methylimidazole, N,N-dimethylbenzylamine, N,N-dimethylbutylamine, N,N-dimethylallylamine, 1,4-diazabicyclo[2,2,2]octane, N,N-dimethyl-4-aminopyridine (DMAP), and N-methylpiperidine, but the present invention is not limited thereto.

<Process 2-2-1>

Process 2-2-1 is a process in which the first composition obtained in Process 1-2-1 and a second composition containing an amine having at least one carboxyl group and water are mixed, and the mixed acid anhydride is reacted with the amine to produce an amide.

In Process 2-2-1 in the method for producing an amide according to the embodiment, a mixed acid anhydride represented by the following General Formula (II) and an amine represented by the following General Formula (VI) are reacted to obtain an amide represented by the following General Formula (VII). 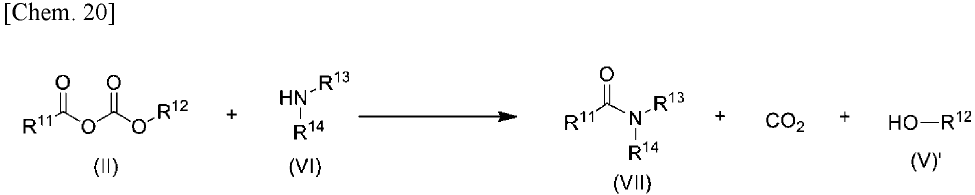 (R¹¹ in Formula (VI) and Formula (VII) has the same meaning as R¹¹ in Formula (II), R¹² in Formula (V)' has the same meaning as R¹¹ in Formula (II), and R¹³ and R¹⁴ in Formula (VI) and Formula (VII) each independently represent a hydrogen atom or a monovalent organic group)

According to the method for producing an amide of the embodiment, it is possible to provide a method for producing an amide, which has favorable reaction efficiency and is less likely to cause epimer production of an amide.

### <Reaction conditions and the like>

In the present embodiment, the amount of each compound used during the reactions in Process 1 to Process 2 may be appropriately adjusted according to a desired reaction in consideration of the types of these compounds.

A molar equivalent ratio (activated carboxylic acid:amine) between the activated carboxylic acid and the amine in the reaction system may be 10:1 to 1/10:1, 5:1 to 1/5:1, or 3:1 to 1/3:1. According to the method for producing an amide of the embodiment, even if a relatively small amount of an amine, which is close to an equivalent amount, is reacted with the carboxylic acid, it is possible to produce an amide with high efficiency.

In the present embodiment, the reaction time for each process may be appropriately adjusted according to other conditions such as the reaction temperature. As an example, the reaction time in Process 1 may be 0.5 seconds to 30 minutes, 1 second to 5 minutes, or 3 seconds to 1 minute. The reaction time in Process 2 may be 1 second to 60 minutes, 3 seconds to 30 minutes, or 5 seconds to 1 minute.

When the mixture containing the first composition and the second composition or the second composition contains a base (B), the molar equivalent ratio (amine:base) between the amine and the base (B) in the reaction system may be 10:1 to 1/10:1, 5:1 to 1/5:1, or 3:1 to 1/3:1.

In the present embodiment, the temperature (reaction temperature) during the reactions in Process 1 to Process 2 may be appropriately adjusted depending on the type of compounds used in Processes 1 and 2. As an example, the reaction temperature is preferably in a range of 0 to 100°C and more preferably in a range of 10 to 50°C.

In the present embodiment, in the reactions in Process 1 to Process 2, the reaction system may further contain other compounds that do not correspond to the above exemplified compounds in a range in which amide production can be achieved.

In the present embodiment, the reactions in Process 1 to Process 2 may be performed separately or simultaneously.

In the present embodiment, the coupling yield of the carboxylic acid and the amine is preferably 70% or more, more preferably 75% or more, still more preferably 80% or more, and particularly preferably 85% or more.

In the present embodiment, the epimer production rate of a desired amide is preferably less than 8%, more preferably less than 5%, still more preferably less than 1%, yet more preferably less than 0.5%, and particularly preferably less than 0.2%.

In the method for producing an amide according to the embodiment described above, the presence and structure of the product can be confirmed by measuring the spectrum obtained by analysis through NMR, IR, mass spectrometry, or the like, or elemental analysis or the like. In addition, as necessary, the product may be purified and can be purified by a purification method such as distillation, extraction, recrystallization, and column chromatography.

### «Method for producing peptide»

In the method for producing an amide according to the embodiment, when the carboxylic acid is an amino acid or an amino acid derivative, and the amine is an amino acid or an amino acid derivative, peptides or proteins can be synthesized. The method for producing an amide includes a method for producing peptides or proteins.

For example, the amide obtained in Process 2 is used as a carboxylic acid in Process 1, after Processes 1 and 2, Processes 1 and 2 are additionally repeated, and a polypeptide chain can be extended.

That is, the carboxylic acid also includes a polypeptide or its derivatives, and the amine also includes a polypeptide or its derivatives. The amino acid or amino acid derivative (carboxylic acid) according to the embodiment also includes an amino acid or an amino acid derivative (carboxylic acid) positioned at the C-terminal as a structural unit of the polypeptide. The amino acid or amino acid derivative (amine) according to the embodiment also includes an amino acid or an amino acid derivative (amine) positioned at the N-terminal as a structural unit of the polypeptide. In this manner, the method for producing an amide according to the embodiment is suitable as a method for producing peptides or proteins.

### «Distribution system reaction device»

The method for producing an amide according to the embodiment can be performed using a distribution system reaction device. A distribution system reaction device including flow paths for transporting a fluid containing raw materials or an intermediate used in the reaction in the method for producing an amide according to the embodiment and a mixing machine for mixing the fluid may be exemplified. Regarding use of the distribution system reaction device, for example, mixing of the first composition and the second composition may be performed in the distribution system reaction device. Regarding the mixing, mixing in Process 2 may be exemplified.

In addition, mixing for obtaining a first composition may be performed in the distribution system reaction device. That is, mixing for obtaining a carboxylic acid active species may be performed in the distribution system reaction device. Regarding the mixing, mixing in Process 1 may be exemplified.

For example, the mixing an organic solvent, a first carboxylic acid, a second carboxylic acid, and phosgene or a phosgene equivalent that decomposes in a reaction system and produces phosgene in Process 1-1 may be performed in the distribution system reaction device.

In addition, for example, the mixing an organic solvent, a carboxylic acid, and an activation agent in Process 1-2 may be performed in the distribution system reaction device.

In addition, for example, the mixing an organic solvent, a carboxylic acid, and a halogenated formate ester in Process 1-2-1 may be performed in the distribution system reaction device.

In addition, for example, the mixing an organic solvent, a carboxylic acid, a halogenated formate ester, and a second base in Process 1-2-1 may be performed in the distribution system reaction device.

Here, the method for producing an amide according to the embodiment is not limited to the method that is performed using the distribution system reaction device. For example, a batch container having a small volume and a high stirring speed may be used.

The volume of the mixing part of the batch container may be 1 to 100 mL or 5 to 50 mL.

Hereinafter, a form of a distribution system reaction device according to an embodiment and an example of a method for producing an amide according to the second embodiment using the same will be described with reference to Fig. 1.

Fig. 1 is a schematic view showing a schematic configuration of a distribution system reaction device 1. The distribution system reaction device 1 includes a tank 11 in which a first liquid is accommodated, a tank 12 in which a second liquid is accommodated, and a tank 13 in which a third liquid is accommodated.

As an example, the first liquid may contain an organic solvent and a carboxylic acid, the second liquid may contain an organic solvent and a halogenated formate ester, and the third liquid may contain water and an amine. As another example, the first liquid may contain an organic solvent, a carboxylic acid and a second base that activates a halogenated formate ester, the second liquid may contain an organic solvent and a halogenated formate ester, and the third liquid may contain a base, water and an amine. As a more specific example, as shown in Fig. 1, the first liquid contains acetonitrile (organic solvent), a carboxylic acid, N-methylmorpholine (second base) and DIEA, the second liquid contains acetonitrile (organic solvent), and isobutyl chloroformate (activation agent), and the third liquid contains a NaOH aqueous solution (NaOH and water), and an amine.

The amine used here may be an unprotected amino acid in which functional groups in the side chain are protected, and amino groups and carboxyl groups forming the main chain part of the peptide are not protected or modified, or an unprotected amino acid that is not protected and modified at any of sites of amino groups, carboxyl groups, and side chains.

Regarding use of the distribution system reaction device, for example, a mixture containing at least the first liquid and the second liquid may be mixed with the third liquid in the distribution system reaction device, and additionally, the first liquid and the second liquid may be mixed in the distribution system reaction device.

The distribution system reaction device 1 includes flow paths f1, f2, f3, f4, and f5 for transporting a fluid. As an example, the inner diameter of the flow path may be 0.1 to 10 mm or 0.3 to 8 mm.

The distribution system reaction device 1 includes mixing machines 31 and 32 for mixing fluids. As an example, the inner diameter of the flow path inside the mixing machine may be 0.1 to 10 mm or 0.3 to 8 mm. Examples of mixing machines include a static mixer having no drive unit. A drive unit is a unit that receives power and moves. Examples of static mixers include a T-shaped mixer and a V-shaped mixer, and a V-shaped mixer is preferable in consideration of mixing efficiency.

In particular, it is preferable to use a V-shaped mixer for mixing the first composition and the second composition.

The inner diameter of the flow path can be a diameter of the inner portion (a portion through which a fluid passes) of the flow path in the cross section of the flow path in a direction perpendicular to the length direction of the flow path. When the shape of the inner portion of the flow path is not a perfect circle, the inner diameter of the flow path can be a diameter when the shape of the inner portion of the flow path is converted into a perfect circle based on the area.

As an example, the tanks 11, 12, 13, and 14, the mixing machines 31 and 32 and the flow paths f1, f2, f3, f4, and f5 are formed of a resin such as a plastic or an elastomer, a glass material, a metal, a ceramic, or the like.

The tank 11 is connected to a pump 21, and the first liquid accommodated in the tank 11 moves through the flow path f1 due to an operation of the pump 21 and flows into the mixing machine 31. The tank 12 is connected to a pump 22, and the second liquid accommodated in the tank 12 moves through the flow path f2 due to an operation of the pump 22 and flows into the mixing machine 31. Then, the first liquid and the second liquid are mixed by the mixing machine 31 to form a first mixed liquid (first composition), and the first mixed liquid is sent to the flow path f4. In a procedure after this mixing, carboxylic acids contained in the first liquid and isopropyl chloroformate contained in the second liquid are dehydrated and condensed to obtain a mixed acid anhydride (Process 1-2-1 in the method for producing an amide). The first mixed liquid (first composition) containing the obtained acid anhydride flows into the mixing machine 32.

On the other hand, the tank 13 is connected to a pump 23, the liquid (second composition) accommodated in the tank 13 moves through the flow path f3 due to an operation of the pump 23, flows into the mixing machine 32, and is mixed with the first mixed liquid (first composition) to form a second mixed liquid, and the second mixed liquid is sent to the flow path f5. In a procedure after this mixing, the mixed acid anhydride obtained in Process 1-2-1 reacts with the amine contained in the third liquid to obtain an amide (Process 2-2-1 in the method for producing an amide). The second mixed liquid containing the produced amide is stored in a tank 14.

In the present embodiment, in order to improve the yield, the mixing speed at which the first composition and the second composition are mixed is preferably 2 mL/min or more and more preferably 3 mL/min or more and 20 mL/min or less at a liquid feeding rate of each composition.

In the example of the distribution system reaction device shown here, the liquid feeding rate of the first liquid is preferably 1.2 mL/min or more, and more preferably 2 mL/min or more. The liquid feeding rate of the second liquid is preferably 2 mL/min or more and more preferably 3 mL/min or more. The liquid feeding rate of the third liquid is preferably 2 mL/min or more and more preferably 3 mL/min or more.

The upper limit value of the liquid feeding rate of the first composition, the second composition, the first liquid, the second liquid, and the third liquid in the distribution system reaction device is not particularly limited, and as an example, it may be 20 mL/min or less or 10 mL/min or less.

According to the distribution system reaction device 1 of the embodiment, the efficiency of mixing the first composition and the second composition is high, the reaction after mixing the first composition containing an organic solvent and the second composition containing water is favorable, and the yield of a desired product can be improved.

According to the distribution system reaction device 1 of the embodiment, it is possible to increase an area for performing heat exchange per volume of the reaction solution. In addition, it is possible to control the reaction time by the flow rate and the length of the flow path. Therefore, it is possible to precisely control the reaction solution, and as a result, it is possible to minimize the progress of undesired side reactions, and it is possible to improve the yield of the desired product.

Since the carboxylic acid active species obtained in Process 1 has high activity, it is important to control the reaction.

According to the distribution system reaction device 1 of the embodiment, when liquids are continuously distributed through the flow paths, an opportunity for compound collision is improved, the reaction can proceed with higher efficiency, and it is easy to minimize side reactions. For example, since the carboxylic acid active species produced in Process 1 can be immediately reacted with a desired amine, the time during which the carboxylic acid active species is in an activated state can be shortened, and it is possible to reduce a probability of the occurrence of side reactions such as isomerization.

Here, in the distribution system reaction device according to the present embodiment, the form in which liquids are mixed by a mixing machine has been exemplified. However, since liquids can be mixed simply by communicating the flow paths with each other, the distribution system reaction device of the embodiment does not necessarily include the mixing machine.

Here, in the distribution system reaction device exemplified above, the method for producing an amide according to the second embodiment has been exemplified, but the method for producing an amide according to the first embodiment can also be performed in the same manner.

As an example, the first liquid may contain an organic solvent, a first carboxylic acid and a second carboxylic acid, the second liquid may contain an organic solvent and phosgene or a phosgene equivalent that decomposes in a reaction system and produces phosgene, and the third liquid may contain a base, water and an amine. As a more specific example, the first liquid may contain acetonitrile (organic solvent), a carboxylic acid and DIEA, the second liquid may contain acetonitrile (organic solvent) and a triphosgene (phosgene equivalent), and the third liquid may contain a NaOH aqueous solution (NaOH and water) and an amine.

The amine used here may be an unprotected amino acid in which functional groups in the side chain are protected, and amino groups and carboxyl groups forming the main chain part of the peptide are not protected or modified, or an unprotected amino acid that is not protected or modified at all sites of amino groups, carboxyl groups, and side chains.

As shown here, the method for producing an amide according to the embodiment can be performed by a liquid phase method. For example, a current mainstream method for producing peptides (amides) is a solid phase method, and peptides in a solid phase may be synthesized. On the other hand, the liquid phase method is suitable for large-scale synthesis, and has favorable reactivity because the degree of freedom of molecules is high. The liquid phase method is also effective in reacting with an amine having low reactivity.

Here, in the distribution system reaction device according to the present embodiment, 5 types of compounds to be reacted are separately accommodated in three tanks. However, for example, the compounds may be accommodated in a total of 5 separate tanks and mixed sequentially.

The substances described above may be in the form of ions, salts or complexes thereof as long as the effects of the present invention can be exhibited.

### [Examples]

While the present invention will be described below in more detail with reference to examples, the present invention is not limited to the following examples.

### <Example 1>

### [Raw materials]

Regarding the amino acid used as a carboxylic acid, Fmoc-Cys (Trt)-OH (commercial product) which is cysteine in which the amino group is protected by the Fmoc group and the thiol group is protected by the Trt group was used. Regarding the amino acid used as an amine, H-Phe-OH which is phenylalanine (an unprotected amino acid in which a carboxyl group is not protected or modified) was used.

### [Flow synthesis of acid amide]

A coupling reaction between the amino acid used as a carboxylic acid and the amino acid used as an amine was caused. For the coupling reaction, a distribution system reaction device including a PTFE tube (an inner diameter of 0.8 mm and an outer diameter of 1.59 mm), an SUS tube (an inner diameter of 0.8 mm and an outer diameter of 1.59 mm), SUS fittings, T-shaped mixer, and a V-shaped mixer was used. Three unreacted solutions were separately prepared. The first solution was obtained by dissolving (a concentration of 0.333 mol/dm³) Fmoc-Cys (Trt)-OH used as a carboxylic acid, DIEA, and N-methylmorpholine in 2.06 mL of acetonitrile. The second solution was obtained by dissolving (a concentration of 0.240 mol/dm³) isobutyl chloroformate in 4.36 mL of acetonitrile. The third solution was obtained by dissolving (amino acid concentration of 0.340 mol/dm³) H-Phe-OH used as an amine in 4.25 mL of a NaOH aqueous solution (a mixture containing 1.53 mL of a 1 M NaOH aqueous solution and 2.72 mL of water). The ratio of each molar concentration in the distribution system reaction device was 1.7 for H-Phe-OH (amine), 1.0 for DIEA, 1.0 for N-methylmorpholine, 1.2 for isobutyl chloroformate, and 1.7 for NaOH with respect to 1.0 of Fmoc-Cys (Trt)-OH (carboxylic acid).

In order to perform coupling in the distribution system reaction device, first, the first solution and the second solution were mixed in a T-shaped mixer and reacted in the distribution system reaction device for 5 seconds to obtain a mixed acid anhydride. Immediately thereafter, a reaction solution containing the mixed acid anhydride and the third solution were mixed using a new V-shaped mixer and reacted in the distribution system reaction device for 10 seconds. All of these reactions were performed at 20°C, and 10 seconds was set as a time for heat exchange before the unreacted solution reached the mixer. Various solutions were discharged using a syringe pump. The flow rate of each pump was 1.2 mL/min for the first solution, 2.0 mL/min for the second solution, and 2.0 mL/min for the third solution.

The reaction of Process 1 in the method for producing an amide in Example 1 is shown below. 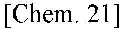 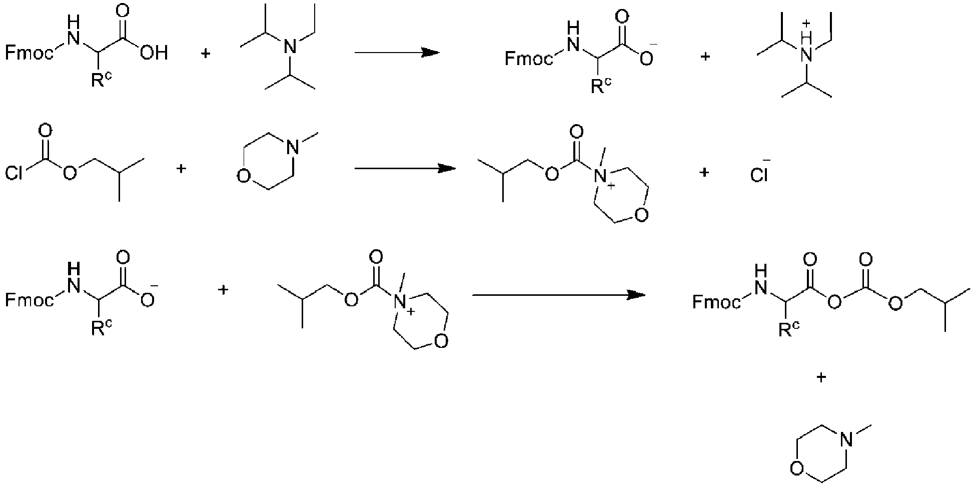 (in the formula, R^{c} represents a cysteine side chain)

The reaction of Process 2 in the method for producing an amide in Example 1 is shown below. 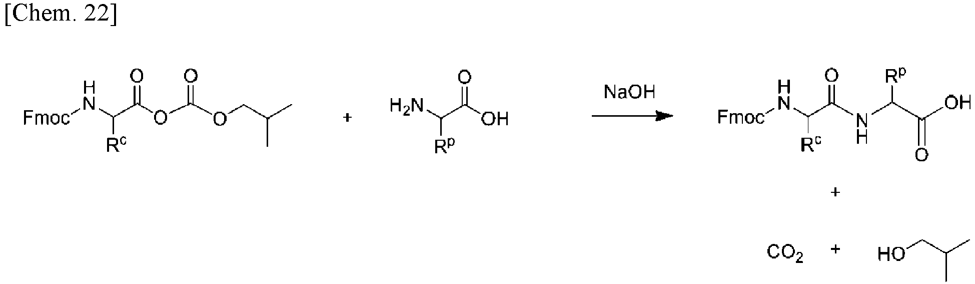 (in the formula, R^{c} represents a cysteine side chain, and R^{p} represents a phenylalanine side chain)

### [Analysis method]

The product after the reaction was isolated by GPC and then identified by NMR analysis.

The yield of the desired product was calculated from the weight of the isolated and purified desired product. That is, the molar equivalent ratio of the carboxylic acid was 1.0, and the coupling ratio of the carboxylic acid was calculated from the weight of the isolated dipeptide.

For the epimer production rate, isomers of isolated and purified amides were separated by HPLC, and the epimer production rate was calculated from the area ratio of the UV absorption intensities of the desired amide and the epimer.

### [Results]

NMR data of the obtained dipeptide is shown below. ¹H NMR (400 MHz, CDCl₃): δ 8.55 (bs, 1 H) 7.71 (dd, J = 9.7, 6.0 Hz, 2 H) 7.52 (t, J = 6.2 Hz, 2 H) 7.34 (m, 8 H) 7.20 (m, 12 H) 7.05 (m, 4 H) 6.51 (d, J = 6.1 Hz, 1 H) 5.20 (d, J = 6.0 Hz, 1 H) 4.74 (dd, J = 5.3 Hz, 5.0 Hz, 1 H) 4.36 (t,J = 5.4 Hz, 1 H) 4.26 (t, J = 5.6 Hz, 1 H) 4.13 (t, J = 5.5 Hz, 1 H) 3.72 (d, J= 4.8 Hz, 1 H) 3.11 (dd, J = 7.0 Hz, 4.0 Hz, 1 H) 2.96 (dd, J = 6.1 Hz, 5.1 Hz, 1H) 2.59 (d, J = 6.3 Hz, 2 H)

As a result of analyzing the product after the reaction, the dipeptide that was the desired product had a coupling yield of 82%. In addition, the area ratio of the UV absorption intensity acquired by HPLC was 99.9% or more for the desired product and the detection limit or less for the epimer, and the epimer production rate was the detection limit or less by HPLC.

### <Example 2>

### [Raw materials]

Regarding the amino acid used as a carboxylic acid, Z-Phg-OH (commercial product) which is phenylglycine in which the amino group is protected by the Z group was used. Regarding the amino acid used as an amine, H-Phe-OH which is phenylalanine (an unprotected amino acid in which a carboxyl group is not protected or modified) was used.

### [Flow synthesis of acid amide]

A coupling reaction between the amino acid used as a carboxylic acid and the amino acid used as an amine was caused. For the coupling reaction, a distribution system reaction device including a PTFE tube (an inner diameter of 0.8 mm and an outer diameter of 1.59 mm), an SUS tube (an inner diameter of 0.8 mm and an outer diameter of 1.59 mm), SUS fittings, a T-shaped mixer, and a V-shaped mixer was used. Three unreacted solutions were separately prepared. The first solution was obtained by dissolving (a concentration of 0.333 M) Z-Phg-OH used as a carboxylic acid, DIEA, and N-methylmorpholine in 3.2 mL of acetonitrile. The second solution was obtained by dissolving (a concentration of 0.240 mol/dm³) isobutyl chloroformate in 6.0 mL of acetonitrile. The third solution was obtained by dissolving (amino acid concentration of 0.340 mol/dm³) H-Phe-OH used as an amine in 5.82 mL of a NaOH aqueous solution (a mixture containing 2.1 mL of a 1 mol/dm³ NaOH aqueous solution and 3.72 mL of water). The ratio of each molar concentration in the distribution system reaction device was 1.7 for H-Phe-OH (amine), 1.0 for DIEA, 1.0 for N-methylmorpholine, 1.2 for isobutyl chloroformate, and 1.7 for NaOH with respect to 1.0 of Z-Phg-OH (carboxylic acid).

In order to perform coupling in the distribution system reaction device, first, the first solution and the second solution were mixed in a T-shaped mixer and reacted in the distribution system reaction device for 5 seconds to obtain a mixed acid anhydride. Immediately thereafter, a reaction solution containing the mixed acid anhydride and the third solution were mixed using a new V-shaped mixer and reacted in the distribution system reaction device for 10 seconds. All of these reactions were performed at 20°C, and 10 seconds was set as a time for heat exchange before the unreacted solution reached the mixer. Various solutions were discharged using a syringe pump. The flow rate of each pump was 1.2 mL/min for the first solution, 2.0 mL/min for the second solution, and 2.0 mL/min for the third solution.

### [Analysis method]

The product after the reaction was isolated through column chromatography and then identified by NMR analysis.

The yield of the desired product was calculated from the weight of the isolated and purified desired product. That is, the molar equivalent ratio of the carboxylic acid was 1.0, and the coupling ratio of the carboxylic acid was calculated from the weight of the isolated dipeptide.

For the epimer production rate, isomers of isolated and purified amides were separated by HPLC, and the epimer production rate was calculated from the area ratio of the UV absorption intensities of the desired amide and the epimer.

### [Results]

NMR data of the obtained dipeptide is shown below. ¹H NMR (400 MHz, MeOD+CDCl₃): δ 7.30-7.11 (m, 15 H) 5.31 (s, 1 H) 5.06 (dd, J = 12.3 Hz, 5.4 Hz, 2 H) 4.71 (t, J = 6.9 Hz, 1 H) 3.19 (dd, J = 8.2 Hz, 5.3 Hz, 1 H) 3.01 (dd, J = 7.3 Hz, 6.4 Hz, 1 H)

As a result of analyzing the product after the reaction, the dipeptide that was the desired product had a coupling yield of 88%. In addition, the area ratio of the UV absorption intensity acquired by HPLC was 99.9% or more for the desired product and the detection limit or less for the epimer, and the epimer production rate was the detection limit or less by HPLC.

### <Example 3>

### [Raw materials]

Regarding the peptide used as a carboxylic acid, Boc-Ala-Ser(Bzl)-OH (synthesized from a commercial product in the distribution system reaction device) which is a dipeptide of alanine in which the amino group is protected by the Boc group and serine in which the hydroxy group is protected by the Bzl group was used. Regarding the amino acid used as an amine, H-Phe-OH which is phenylalanine (an unprotected amino acid in which a carboxyl group is not protected or modified) was used.

### [Flow synthesis of acid amide]

A coupling reaction between the dipeptide used as a carboxylic acid and the amino acid used as an amine was caused. For the coupling reaction, a distribution system reaction device including a PTFE tube (an inner diameter of 0.8 mm and an outer diameter of 1.59 mm), an SUS tube (an inner diameter of 0.8 mm and an outer diameter of 1.59 mm), SUS fittings, a T-shaped mixer, and a V-shaped mixer was used. Three unreacted solutions were separately prepared. The first solution was obtained by dissolving (a concentration of 0.333 mol/dm³) Boc-Ala-Ser(Bzl)-OH used as a carboxylic acid, DIEA, and N-methylmorpholine in 3.1 mL of acetonitrile. The second solution was obtained by dissolving (a concentration of 0.240 mol/dm³) isobutyl chloroformate in 6.0 mL of acetonitrile. The third solution was obtained by dissolving (amino acid concentration of 0.340 mol/dm³) H-Phe-OH used as an amine in 5.82 mL of a mixed solvent in which the acetonitrile/NaOH aqueous solution (a mixture containing 2.10 mL of a 1 M NaOH aqueous solution and 3.72 mL of acetonitrile) were mixed at a volume ratio of 2:1 (acetonitrile:NaOH aqueous solution). The ratio of each molar concentration in the flow reaction system was 1.7 for H-Phe-OH, 1.0 for DIEA, 1.0 for N-methylmorpholine, 1.2 for isobutyl chloroformate, and 1.7 for NaOH with respect to 1.0 of Boc-Ala-Ser(Bzl)-OH.

In order to perform coupling in the distribution system reaction device, first, the first solution and the second solution were mixed in a T-shaped mixer and reacted in the distribution system reaction device for 5 seconds to obtain a mixed acid anhydride. Immediately thereafter, a reaction solution containing the mixed acid anhydride and the third solution were mixed using a new V-shaped mixer and reacted in the distribution system reaction device for 10 seconds. All of these reactions were performed at 20°C, and 10 seconds was set as a time for heat exchange before the unreacted solution reached the mixer. Various solutions were discharged using a syringe pump. The flow rate of each pump was 1.2 mL/min for the first solution, 2.0 mL/min for the second solution, and 2.0 mL/min for the third solution.

### [Analysis method]

• The product after the reaction was isolated by recrystallization and then identified by NMR analysis.

The yield of the desired product was calculated from the weight of the isolated and purified desired product. That is, the molar equivalent ratio of the carboxylic acid was 1.0, and the coupling ratio of the carboxylic acid was calculated from the weight of the isolated tripeptide.

For the epimer production rate, isomers of isolated and purified amides were separated by HPLC, and the epimer production rate was calculated from the area ratio of the UV absorption intensities of the desired amide and the epimer.

### [Results]

NMR data of the obtained dipeptide is shown below. ¹H NMR (400 MHz, MeOD+CDCl₃): δ 7.97 (s, 1 H) 7.35-7.14 (m, 9 H) 4.71-4.64 (m, 1 H) 4.55 (bs, 1 H) 4.52 (d, J = 2.8 Hz, 2 H) 4.09 (bd, J = 6.7 Hz, 1 H) 3.75 (dd, J = 5.1 Hz, 4.5 Hz, 1 H) 3.67 (dd, J = 5.1 Hz, 4.6 Hz, 1 H) 3.18 (dd, J = 8.5 Hz, 5.4 Hz, 1 H) 3.03 (dd, J = 7.4 Hz, 6.5 Hz, 1 H) 1.42 (s, 9 H) 1.27 (d, J =7.2 Hz, 3 H)

As a result of analyzing the product after the reaction, the tripeptide that was the desired product had a coupling yield of 77%. In addition, the area ratio of the UV absorption intensity acquired by crude HPLC analysis was 99.9% for the desired product and 0.1% for the epimer, and the epimer production rate was about 0.1%.

According to the methods shown in Examples 1 to 3, although an unprotected amino acid (amine having at least one carboxyl group) was used as a raw material, the molar equivalent ratio of the amine to the carboxylic acid was 1.7:1, and the reaction time was as short as 10 seconds, it was possible to achieve a high coupling yield and reduce the epimer production rate to a very low level.

While embodiments of the invention have been described above in detail with reference to chemical formulae and drawings, configurations and combinations thereof in the embodiments are only examples, and configurations can be added, omitted, and replaced and other modifications can be made without departing from the spirit and scope of the present invention. In addition, the present invention is not limited to the embodiments, but is limited only by the scope of claims.

### [Reference Signs List]

1 Distribution system reaction device
11, 12, 13, 14 Tank
21, 22, 23 Pump
31, 32 Mixing machine
f1, f2, f3, f4, f5 Flow path

## Claims

1. A method for producing an amide, comprising
mixing a first composition containing a carboxylic acid or a carboxylic acid active species, and an organic solvent and a second composition containing an amine having at least one carboxyl group and water and causing the carboxylic acid or the carboxylic acid active species to react with the amine to obtain an amide.

2. The method for producing an amide according to claim 1, comprising
before the mixing, activating the carboxylic acid to obtain the carboxylic acid active species.

3. The method for producing an amide according to claim 1 or 2, comprising
mixing a first composition containing a product obtained by reacting a mixture obtained by mixing a carboxylic acid and an activation agent, and an organic solvent and a second composition containing an amine having at least one carboxyl group and water and causing the product to react with the amine to obtain an amide.

4. The method for producing an amide according to claim 3, comprising
before the mixing, reacting a mixture obtained by mixing a carboxylic acid and an activation agent.

5. The method for producing an amide according to any one of claims 1 to 4,
wherein the first composition contains the carboxylic acid active species, and the carboxylic acid active species is any one or more selected from the group consisting of a mixed acid anhydride, a mixed carbonate anhydride, an acid azide, an acid halide, a benzotriazole amide, a pentafluorophenol ester, a p-nitrophenol ester, and a succinimide ester.

6. The method for producing an amide according to claim 5,
wherein the mixed acid anhydride is synthesized by any one or more selected from the group consisting of isopropyl chloroformate, isobutyl chloroformate, ethyl chloroformate, 2,4-dimethyl-3-pentyl chloroformate, isopropyl bromoformate, isobutyl bromoformate, ethyl bromoformate, and 2,4-dimethyl-3-pentyl bromoformate.

7. The method for producing an amide according to any one of claims 3 to 6,
wherein the activation agent is a halogenated formate ester, and
wherein the first composition contains a product obtained by reacting a mixture obtained by mixing a carboxylic acid, the halogenated formate ester, and a second base that activates the halogenated formate ester, and an organic solvent.

8. The method for producing an amide according to claim 7,
wherein the second base that activates the halogenated formate ester is any one or more selected from the group consisting of tertiary amines, 4-methylmorpholine, pyridine, pyridine derivatives, imidazole, imidazole derivatives and 1,4-diazabicyclo[2,2,2]octane.

9. The method for producing an amide according to claim 1 or 2, comprising
mixing a first composition containing a product obtained by reacting a mixture obtained by mixing a first carboxylic acid and a second carboxylic acid, and an organic solvent, and a second composition containing an amine having at least one carboxyl group and water, and causing the product to react with the amine to obtain an amide.

10. The method for producing an amide according to claim 9, comprising
before the mixing, reacting a mixture obtained by mixing a first carboxylic acid and a second carboxylic acid.

11. The method for producing an amide according to claim 10,
wherein the first composition contains a product obtained by mixing the first carboxylic acid, the second carboxylic acid, and phosgene or a phosgene equivalent that decomposes in a reaction system and produces phosgene and dehydrating and condensing the carboxylic acids, and the organic solvent.

12. The method for producing an amide according to any one of claims 1 to 11,
wherein the carboxylic acid is an amino acid or an amino acid derivative.

13. The method for producing an amide according to any one of claims 1 to 12,
wherein the amine is an amino acid or an amino acid derivative.

14. The method for producing an amide according to any one of claims 1 to 13,
wherein the amine is an unprotected amino acid in which functional groups in the side chain are protected, and amino groups and carboxyl groups are not protected or modified.

15. The method for producing an amide according to any one of claims 12 to 14,
wherein the amino acids or amino acid derivatives are 20 types of amino acids or derivatives thereof that constitute proteins and are encoded by genetic information.

16. The method for producing an amide according to any one of claims 1 to 15,
wherein the first composition and the second composition are mixed in a distribution system reaction device.

17. The method for producing an amide according to claim 16,
wherein, in the distribution system reaction device, a liquid feeding rate of the first composition is 2 mL/min or more, and a liquid feeding rate of the second composition is 2 mL/min or more.

18. The method for producing an amide according to any one of claims 1 to 17,
wherein the second composition or a mixture containing the first composition and the second composition contains a base.

19. The method for producing an amide according to any one of claims 1 to 18,
wherein, in the second composition, a proportion of the content of water with respect to 100 volume% of the component corresponding to the solvent is 10 to 100 volume%.

20. The method for producing an amide according to any one of claims 1 to 19,
wherein the organic solvent is compatible with water.
